# EUROPEAN PATENT SPECIFICATION

(11) **EP 4 120 914 B1**
(45) Date of publication and mention of the grant of the patent: **07.05.2025**
(21) Application number: 21714274.4
(22) Date of filing: 18.03.2021
(51) Int. Cl.: A61B 5/282, A61B 5/344, A61B 5/00

(54) **FETAL HEART RATE MONITORING DEVICE AND METHOD OF CONTROLLING THEREOF**
VORRICHTUNG ZUR ÜBERWACHUNG DER FÖTUSHERZFREQUENZ UND VERFAHREN ZUR STEUERUNG DAVON
DISPOSITIF DE SURVEILLANCE DU RYTHME CARDIAQUE FOETAL ET SON PROCÉDÉ DE COMMANDE

(30) Priority: 19.03.2020 EP 20164220
(43) Date of publication of application: 25.01.2023
(73) Proprietor: Nederlandse Organisatie voor toegepast-natuurwetenschappelijk Onderzoek TNO, 2595 DA 's-Gravenhage (NL)
(72) Inventor: PHOELICH, Pieter Franciscus, 2595 DA 's-Gravenhage (NL); VAN DONGEN, Pauline Louisette, 2595 DA 's-Gravenhage (NL)
(74) Representative: V.O.
(86) International application number: PCT/NL2021/050185
(87) International publication number: WO 2021/187984

(56) References cited:
- WO-A1-2018/134844
- WO-A2-2017/071785
- US-A1- 2007 191 728
- US-A1- 2016 317 088
- US-A1- 2016 374 608
- US-A1- 2018 184 920

## Description

### Field of the invention

The present invention is directed at a fetal heart rate monitoring device for monitoring a heart rate of a fetus as defined in independent claim 1.

The invention is further directed at a method of controlling a fetal heart rate monitoring device as defined in independent claim 13.

### Background

Typically, if an expecting mother wants to experience the heart rate of her unborn baby, like an audible signal in an ultrasound echo, she needs to visit a professional healthcare facility. Alternatively or additionally, she can buy a commercially available fetal heartrate detection system that are nowadays available on the market. Such a system, which may be a handheld, belt or self-adhering bandage intended for fetal monitoring, requires proper positioning in order to obtain a (sufficiently strong) fetal heart rate signal. For professional healthcare employees this is a daily duty. Mothers to-be, however, may find this a more challenging task. Improper use of handheld, smart bandages or belts may then lead to confusion and dissatisfaction, or even worse, panic that something may be wrong if no proper signal can be achieved.

US patent application no. US 2007/0191728 relates to an intrapartum monitor patch. US 2016/374608 is directed at systems, devices, and methods for tracking abdominal orientation and activity. Furthermore, International application no. WO 2018/134844 relates to a portable auscultation device.

### Summary of the invention

It is an object of the present invention to overcome the abovementioned disadvantages of conventional fetal heartrate detection systems, and to provide a fetal heart rate monitoring device that can be used also by unexperienced users to obtain a fetal heart rate signal from their unborn child.

To this end, there is provided herewith a fetal heart rate monitoring device for monitoring a heart rate of a fetus, the device comprising: a carrier suitable for being attached to a human body; at least one fetal electrocardiographic sensor mounted on the carrier and configured for providing an output signal; at least one reference sensor for providing a reference signal for filtering the output signal to enable identifying a fetal heartrate therefrom; wherein the device further comprises: an actuator arrangement comprising a plurality of actuators mounted on the carrier and distributed in an area around the fetal electrocardiographic sensor, wherein each actuator is configured for providing a haptic signal to the human body; and a controller communicatively connected to the at least one fetal electrocardiographic sensor for receiving the output signal, wherein the controller is configured for determining a signal strength from the received output signal, wherein the controller is arranged for controlling each of the plurality of actuators for providing a haptic feedback signal to the human body dependent on the received output signal wherein the controller is configured for providing the haptic feedback signal dependent on the determined signal strength.

The fetal heart rate monitoring device of the present invention is able, using the reference sensor in combination with the at least one fetal electrocardiographic sensor, to distinguish the relatively weak fetal heart rate signal from the background signal, and to provide direct haptic feedback to the user via the actuators. For example, the user may place the carrier of the fetal heart rate monitoring device on her belly and receive a haptic sensation via her skin in case the output signal contains the fetal heart rate signal. For example, the providing of a direct and real time haptic feedback signal via the skin makes it much more easy for a user to find the correct location where the fetal heart rate signal can be picked up. Alternatively, as we will further explain below, in certain embodiments direction may be provided via the haptic signal to move to the correct location where the fetal heart rate signal will be received stronger or is at its maximum strength.

Furthermore, the use of a haptic signal provided via the actuators, makes the use of external feedback mechanisms - such as sound generators or Bluetooth connections to a mobile phone or other external device - obsolete. The monitoring device may therefore, in accordance with some embodiments, be completely self-contained, e.g. including a power supply (e.g. battery, solar panel, or charging element). Although a connection with an external element for these purposes is not needed, as may be appreciated any plugs, sockets or other means to facilitate a connection with an external device may in some embodiment nevertheless be present if deemed advantageous or desired for any additional purpose. For example, such a connection may be used for transferring time dependent fetal heart rate data that may be temporarily stored in the device, or to send or receive trigger signals to control certain device functions, or for some other purpose.

In accordance with some embodiments the controller is configured for determining a heart rate frequency from the received output signal, and for providing the haptic feedback signal as a periodic signal having a periodicity corresponding with the determined heart rate frequency. As may be appreciated, the direct haptic feedback, via the skin, of the fetal heart rate in an arbitrary or suitably selected periodic signal - the periodicity of which corresponds with the fetal heart rate frequency determined, will be comforting to the user and strengthen the user's bond with the unborn baby.

According to the invention, the controller is configured for determining a signal strength from the received output signal, and for providing the haptic feedback signal dependent on the determined signal strength. For example, the haptic feedback signal may be intensified in case the signal strength from the output signal (i.e. from the fetal heart rate signal found in the output signal) increases. This further enhances the ability of the user to find the location where the fetal heart rate may best be picked up, and further increases the level of comfort once the fetal heart rate signal has been found and is provided as feedback to the mother via the haptic signal.

In some of these embodiments, the controller is configured for individually controlling each of the actuators for providing the haptic feedback signal, and for selectively adapting for each respective actuator an intensity of the haptic feedback signal provided via the actuator dependent on the determined signal strength. By controlling the (intensity of) the haptic feedback provided by the individual actuators separately, it becomes possible to provide directions to the user via the haptic feedback signal.

For example, in certain embodiments, during a relative motion of the carrier on and across the human body, the controller may receive one or more signal samples of the output signal, wherein the controller is configured for determining a signal strength from each of the signal samples, and wherein the controller is configured for providing, based on the determined signal strength and by selectively controlling one or more of the actuators, an indication of a direction wherein a relative location of the carrier on the human body is to be changed for increasing the determined signal strength. The user, in this embodiment, is led by the haptic signals provided to move the carrier with the fetal electrocardiographic sensor towards a local maximum in received signal strength from the fetal heart rate signal detected. This is done on the basis of collected signal samples that are collected and (temporarily) stored while the carrier is being moved over and across the skin of the human body. The user will thereby be aided to quickly find a good location to pick up the heart rate.

In yet other of these embodiments, the device further comprises one or more auxiliary sensors for providing auxiliary output signals, and the controller is configured for determining an auxiliary signal strength from each of the auxiliary output signals, and for providing, based on the determined auxiliary signal strengths and by selectively controlling one or more of the actuators, an indication of a direction wherein a relative location of the carrier on the human body is to be changed for increasing the determined signal strength of the output signal received from the at least one fetal electrocardiographic sensor. As may be appreciated, instead of or in addition to the embodiment above, wherein history data of signal samples are used to determine a direction wherein the carrier may need to be moved to increase the signal, the auxiliary sensors of the present embodiment enable to provide multiple signal samples simultaneously and thereby enable to determine the direction without requiring history data. Thus, the user in these embodiments, upon placing the carrier on her skin, may immediately be directed towards the direction wherein the signal strength of the fetal heart rate signal increases. This makes it even more easy to find the fetal heart rate signal.

In various embodiments of the invention, the fetal heart rate monitoring device comprises at least four actuators distributed in the area around the at least one fetal electrocardiographic sensor such that at least one of the actuator is located in each quarter of the area. Having a minimum of four actuators, with at least one of them in each quarter of the area around the fetal electrocardiographic sensor, enables to provide simple directions via the haptic feedback signal, such as: up, down, left, and right. The four (or more) actuators with one of them in each quarter are a specific embodiment of the more general class of embodiments below, wherein, the fetal heart rate monitoring device comprises a plurality of actuators such as to comprise at least n actuators, wherein the area around the at least one fetal electrocardiographic sensor comprises an n number of sectors, such that at least one actuator is located in each one of the n number of sectors. For example, eight actuators in eight sectors enable to indicate directions such as: up, up-right, right, down-right, down, down-left, left, or up-left. In the embodiment with four quarters and (at least) four actuators, the directions: up-right, down-right, down-left, or up-left may of course be indicated by operating multiple actuators simultaneously to provide a haptic feedback signal.

In some embodiments, the controller is configured for providing, based on the determined signal strengths, and optionally (if applicable to the embodiment) the auxiliary signal strengths from the auxiliary sensors, an indication of a relative location of the heart of the fetus in the human body by selectively controlling the haptic signal of one or more of the actuators. By adapting the intensities of haptic feedback signal provided by the various actuators relative to each other, the location of the haptic sensation as perceived by the user may be changed. This may be done dependent on a determined expected relative location of the heart of the fetus (i.e. the projection thereof (nearest location) on the skin of the belly). Thereby, the user can sense the fetus movements in the uterus, and knows where about the fetus is located, i.e. in terms of the relative location on her belly.

In some embodiments, the carrier comprises a flat bendable or flexible substrate material. The carrier may be configured for being attached, at least temporarily, to the user's body, e.g. by adhering or otherwise fixing. In various embodiments the carrier is arranged for being adhered to a skin of the human body. In some embodiments, the carrier is provided by a patch for attachment to the human body. In other embodiments, the carrier is provided by a belt or cloth that may be attached around an abdominal region of the human body. In some embodiments, the carrier is arranged for supporting the fetal electrocardiographic sensor such as to be located on the human body on a belly thereof. In particular, optionally, the carrier is arranged for supporting the fetal electrocardiographic sensor such as to be located in at least one of: an umbilical region, a right or left lumbar region, a hypogastrium region, or a right or left iliac region.

In some preferred embodiments, in the fetal heart rate monitoring device, one or more of: the at least one fetal electrocardiographic sensor, the reference sensor, the actuator arrangement, one or more the actuators of the actuator arrangement, one or more electrical connections of the device, or one or more electrodes of the device, are provided by printed electronics. In particular in combination with some of the above embodiments, e.g. the flat and bendable substrate and/or the patch that may be adhered, this provides an embodiment that may be worn underneath the user's clothing such as to provide haptic feedback throughout the day.

In some embodiments, the fetal heart rate monitoring device further comprises at least one ground electrode located in between at least one of the fetal electrocardiographic sensors and the reference sensor. The additional ground electrode further enables to reduce the noise level in the output signal of the fetal electrocardiographic sensor. The ground electrode, however, is certainly not essential to the invention, and the invention may operate without the additional grounding electrode.

In accordance with a seconds aspect, there is provided a method of controlling a fetal heart rate monitoring device for monitoring a heart rate of a fetus located inside a human body, wherein the device comprises a controller, at least one fetal electrocardiographic sensor configured for providing an output signal to the controller, at least one reference sensor for providing a reference signal for filtering the output signal, and a plurality of actuators wherein each actuator is configured for providing a haptic signal to the human body; the method comprising a steps of: receiving, by the controller, the output signal from the at least one fetal electrocardiographic sensor; and providing, by controlling each of the plurality of actuators, a haptic feedback signal to the human body dependent on the received output signal. This method may be applied by a controller in a fetal heart rate monitoring device as described above in accordance with the first aspect.

In some embodiments, the method of the second aspect further comprises obtaining, by the controller, at least one of: one or more signal samples of the output signal of the at least one fetal electrocardiographic sensor, or one or more signal samples of auxiliary output signals provided by auxiliary sensors; determining, by the controller, one or more signals strengths from the signal samples; and providing, by the controller, based on the determined signal strengths and by selectively controlling one or more of the actuators, an indication of a direction wherein a relative location of the carrier on the human body is to be changed for increasing a signal strength of the output signal received from the at least one fetal electrocardiographic sensor.

In some embodiments, the method further comprises: obtaining, by the controller, at least one of: one or more signal samples of the output signal of the at least one fetal electrocardiographic sensor, or one or more signal samples of auxiliary output signals provided by auxiliary sensors; determining, by the controller, one or more signals strengths from the signal samples, and an expected relative location of the heart of the fetus in the human body; and providing an indication of the determined expected relative location of the heart of the fetus in the human body by selectively controlling the haptic signal of one or more of the actuators.

### Brief description of the drawings

The invention will further be elucidated by description of some specific embodiments thereof, making reference to the attached drawings. The detailed description provides examples of possible implementations of the invention, but is not to be regarded as describing the only embodiments falling under the scope. The scope of the invention is defined in the claims, and the description is to be regarded as illustrative without being restrictive on the invention. In the drawings:
Figure 1 schematically illustrates a fetal heart rate monitoring device in accordance with an embodiment of the present invention;
Figure 2 schematically illustrates control electronics for a fetal heart rate monitoring device in accordance with an embodiment of the present invention;
Figure 3 schematically illustrates a fetal heart rate monitoring device in accordance with an embodiment of the present invention in use by a user.

### Detailed description

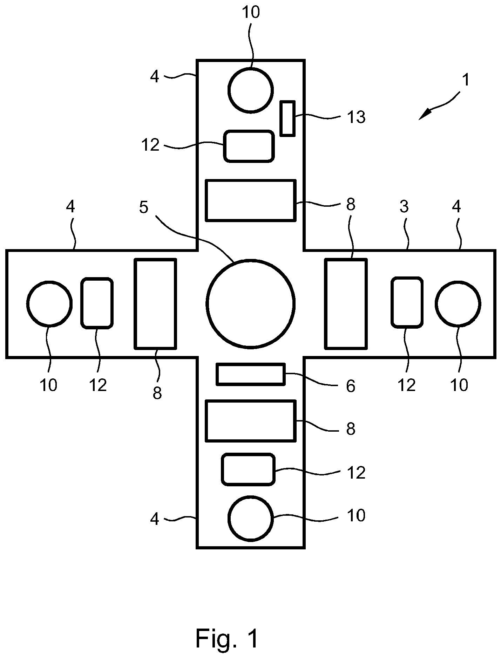

Figure 1 schematically illustrates a fetal heart rate monitoring device 1 in accordance with an embodiment of the present invention. The device comprises a carrier substrate 3 having thereon and integrated therein a plurality of components of the device. The carrier may be any kind of suitable carrier for the application, and suitable for being fixed relative to and in contact with the skin of a user. For example, the carrier 3 may comprise a patch having an adhering side that may be adhered to the skin, or a soft cloth that may be attachable by adhering patch sections or by one or more belts with quick-release connectors, clips, velcro or the like. The carrier 3 is preferably a flat carrier substrate that may be bendable or flexible to follow the contours of the contours of a user's skin. The components on the carrier are preferably provided by printed electronics, which enable the whole to be embodied as a flat arrangement of parts. However, instead of or in addition to printed electronics, these components and any other parts that may be provided by printed electronics may also be provided using other types of flexible electronics, such as copper polyimide. For example, the carrier and its components may consist of functional layers: an arrangement of electrodes to be in contact with the skin, a carrier substrate layer, a printed electronics layer, and a cover layer. This is not essential though, as the skilled reader may appreciate, and certain layers may be integrated into a single layer, or alternatively the arrangement may not be embodied as a flat arrangement at all. For example, non-flat components that may have been selected to reduce the cost of manufacturing or selected because of reliability or accuracy, or for other reasons, may likewise be applied. In the above, although the electrodes are not illustrated, it is to be understood that the electrodes connect to the respective functional components in the upper layers or on the carrier 3 to provide a local connection with the skin. For example, each of the sensors may comprise an electrode (or be connected with an electrode) in touch with the skin to receive input signals. The actuators to be described do not necessarily require an electrode or functional part that is in touch with the skin, as will become apparent below, although of course this is not prohibited and in some cases being in contact with the skin may even be advantageous.

The fetal heart rate monitoring device 1 of figure 1 further comprises a fetal electrocardiographic sensor 5 (fECG sensor). A fECG sensor 5 is configured for sensing the small electrical impulses from the heart fetus and provide an output signal indicative of the sensed signal. This sensing technique is similar to regular ECG sensing, although a fECG is much more sensitive to enable picking up the much weaker cardiac signal from the fetus. Typically, because the fECG also picks up noise and other cardiac signals around (i.e. the cardiac signal of the mother), the fECG signal requires filtering in order to provide a strong enough fetal heart rate signal in the output signal to distinguish this from the disturbance. In the embodiment of figure 1, an optional reference sensor 13 is illustrated. The location of the reference sensor 13 is, in this embodiment, in the upper part of the carrier 3 which is closer to the heart of the mother. The reference sensor 13 is optional in the embodiment in the sense that it is not necessary that this reference sensor is additional to any optional auxiliary sensors present in the design. Here, in the embodiment of figure 1, auxiliary sensors 12 as will be described further down below, are present in the design and may be applied to also provide a reference signal for filtering. Hence, in the embodiment of figure 1, the reference sensor 13 may be a bit redundant and may thus be dispensed with. It has been included in the drawing for explanatory purposes though.

The carrier 3 of figure 1 is embodied as a patch having a central part 2 and a number of extremities 4. At the ends of each extremity 4, the patch bears an actuator 10 such that in total there are four actuators 10. The actuators 10 provide haptic feedback signals, as will be explained. The actuators are controlled using the control electronics 6, and are electrically and communicatively connected therewith. The device 1 further comprises auxiliary sensors 12. The auxiliary sensors 12 are further fetal electrocardiographic sensors providing auxiliary output signals. Furthermore, each of the extremities 4 includes an optional ground electrode 8 in touch with the skin. The ground electrode reduces the background noise in the fECG sensor 5 and the auxiliary sensors 12. The ground electrodes 8 are optional and may be absent in some embodiments. The patch being shown in figure 1 has a cross shape with four arms or extremities 4. The skilled person may appreciate that this shape is merely illustrative, and not essential. The patch may have an arbitrary shape or size, although must be large enough to allow bearing the components and spanning an area that is large enough so that a user is able to distinguish between the haptic feedback signals coming from different actuators 10. Naturally, the patch must be large enough to support all electrodes of the embodiment. Furthermore, the patch may be adhesive or otherwise treated such that it may easily adhere to the skin frequently in order to enable the patch to be removed and placed in a different location on the skin.

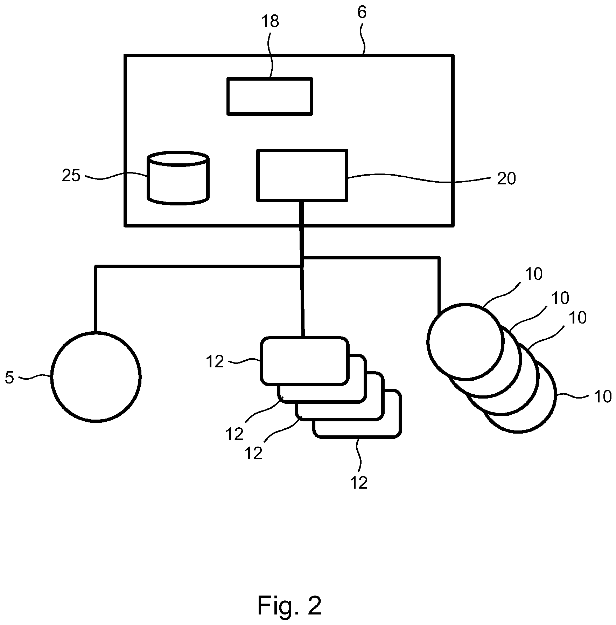

In the device 1, the fECG sensor 5 provides an output signal that includes the fetal heart rate signal. This signal is filtered using the reference electrode 13 to enable distinguishing the fetal heart rate signal out of the disturbances provided by the mothers cardiac signal and noise signals. The control electronics, which are shown in figure 2 and will be explained below, are configured for receiving the output signal from the fECG sensor 5 and, dependent on the distinguished fetal heart rate signal, activate one or more or all of the actuators 10 to provide a haptic feedback signal. The actuators 10 may be configured for providing a vibrational signal, pressure signal or possibly a thermal signal, such as to provide a haptic feedback sensation to the user, i.e. the mother. The actuators 10 may be providing a periodic heart rate signal mimicking the fetal heart rate, i.e. having a periodic frequency that equals the fetal cardiac frequency.

The haptic feedback signal provided by actuators 10 may be provided at a selected position, i.e. such that it is sensed at a certain position, by selectively controlling one or more of the actuators 10. This position dependency may be controlled by the controlling electronics dependent on e.g. the output signal from the fECG sensor 5 or on history data of signal samples that were acquired earlier. For example, the user may have moved the device 1 relative to her body and in contact with the skin, while at the same time signal samples of the output signal of sensor 5 were taken by the controller 20 which were temporarily stored in a buffer or memory 25. Preferably, the auxiliary signals from auxiliary sensors 12 are applied to monitor movements, i.e. changes in the relative position of the device 1 with respect to the user's body. This may be achieved by performing e.g. triangulation on either the mother's cardiac signal (using the mother's heart as fixed reference point in the body) or the fetal cardiac signal (thereby assuming a temporary static position of the fetus while the device 1 is moved relative to the body, before being fixed thereto). The skilled person may appreciate that signals from other fixed references in or on the body may possibly be used in addition or alternatively. In principle, in certain embodiments, although this is somewhat cumbersome and therefore not the most advantageous solution, external signal sources may even be applied to generate a fixed reference signal, e.g. a further patch (separate from the device 1) with an electrode. In embodiments without auxiliary sensors 12, the main output signal from sensor 5 and for example signals from a motion sensor may be applied to either determine a relative position of the device 1 on the user's body or to determine movement data. From this, directional instructions on how to move the device 1 relative to the body 30 may be derived. The above embodiments allow to store, in addition to the signal samples, positional data of the relative position of the device 1 relative to the body or movement data of movements that were made by the device 1 relative to the body.

From the historic signal samples, and e.g. after a signal strength determination by the controller (of the fetal cardiac signal strength), an expected relative location (or projection thereof on the skin of the belly) of the fetal's heart may be determined (e.g. as the location of a local maximum in the fetal's cardiac signal). The haptic feedback signal may be provided at or near this location, by selectively controlling one or more of the actuators 10 near this location, or by providing the haptic feedback signal via each of the actuators with a different signal intensity. Alternatively, the historic data may be used to guide the mother/user to move the patch to an optimal location for obtaining a strong heart rate signal. For example, based on the historic data, the haptic feedback signal via actuators 10 may be provided in a manner to guide the user to move the device 1 to the location where the best (i.e. strongest and/or least disturbed) output signal may be obtained from which the fetal heart rate signal may best be distinguished. For example, with four actuators 10 as depicted in figure 1, directional signals may be given by the control electronics 6 by controlling the operation of actuators 10 selectively to provide the haptic signal via selected ones of the actuators 10. For example, by controlling the upper actuator 10 to provide a haptic feedback signal, the direction 'up' can be given.

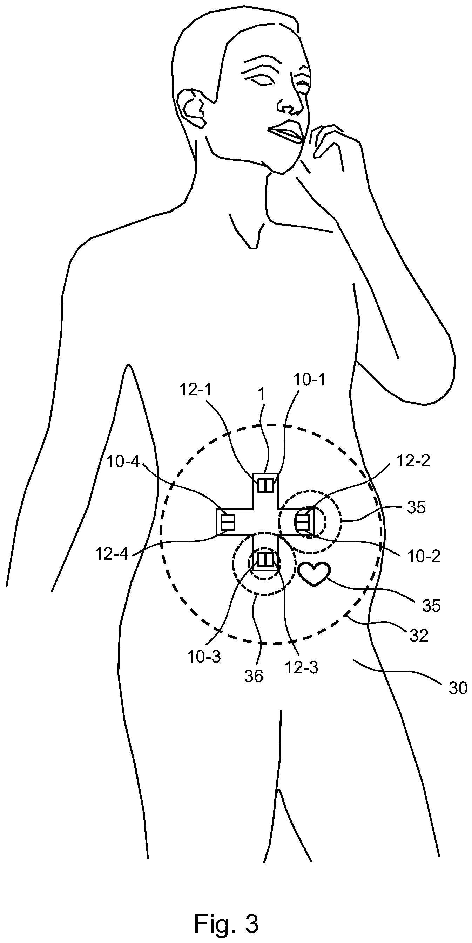

An example of the above guiding method is schematically illustrated in figure 3. In Figure 3, a pregnant mother 30 is schematically illustrated. The contours 32 schematically illustrate a belly of the user 30. In connection herewith, it is to be noted that although the present description frequently refers to the user's belly as a desired location to place the device 1, this is not intended to be interpreted as limiting on the invention. The device may be placed anywhere on the body where a sufficiently strong fetal heart rate signal may be acquired. This may be on the belly, e.g. the abdominal region, such as an umbilical region, a right or left lumbar region, a hypogastrium region, or a right or left iliac region. However, this may likewise be from the side of the abdomen (hypochondriatic, lumbar or iliac regions) or the user's back (e.g. the lumbar or dorsal parts). The device 1 illustrated in figure 3 will guide the user to a location where the fetal cardiac signal may be acquired best. This location is schematically depicted by heart 35, i.e. the assumed fetal heart position.

The device 1 performs fetal heart rate signal measurements using the fetal electrocardiographic sensor 5 and the auxiliary sensors 12-1, 12-2, 12-3 and 12-4. From these measurements it can be determined that the fetal cardiac signal that origins from location 35, is best received via auxiliary sensors 12-2 and 12-3. The actuators 10-1, 10-2, 10-3 and 10-4, which are located close to the auxiliary sensors 12-1 to 12-4, are then operated to provide the haptic feedback signal 36 via actuators 10-2 and 10-3. The user 30 feels these haptic feedback signals 36 and intuitively interprets this as a direction to move the device 1 in the lower-left direction (in figure 3 this is the lower-right direction). Correct placement of the device 1 over position 35, such that the location 35 more or less is located underneath or in the direct vicinity of fetal electrocardiographic sensor 5, will result in the haptic feedback signal 36 being provide through all actuators 10-1 to 10-4 equally. This will tell the user 30 that no further corrections are needed and that she can fix the device relative to the body in that location. When the fetus moves inside the uterus, the location where the haptic feedback is provided will likewise be adapted by the control electronics 6 by changing the intensity of the haptic feedback signal as it is provided by each individual actuator 10-1 to 10-4. Small changes may be indicated by subtle intensity differences between the different actuators 10-1 to 10-4. For example, if the fetus moves down, the intensity of actuator 10-3 may be increased and the intensity of actuator 10-1 may be decreased. If the location of the fetus moves considerably, the actuators 10 located farthest away from the new fetal location 35 may cease providing a haptic signal. For example, a situation as is illustrated in figure 3 may again be obtained, where only actuators 10-2 and 10-3 are operated to provide the haptic feedback signal 36. This will indicate to the user 30 that it may be time to change the location of device 1.

Figure 2 schematically illustrates the control electronics 6 of a device 1 in accordance with an embodiment. The control electronics 6 at least comprises a power supply 18 and a processor 20. The processor 20 may communicatively be connected with an optional memory 25 for storing data, e.g. signal samples and other data, such as positional data or movement data. The processor 20 is further communicatively connected with an electrocardiographic sensor 5 and actuators 10. Furthermore, if present, the processor 20 is further communicatively connected with the auxiliary sensors 12. Additional electronic components may be present but are omitted from the drawing in order to not obscure the presentation and explanation of the invention with unnecessary details. However, to mention some of these additional components that may be present optionally, an accelerometer may for example be applied to guide measurement and recordation of movements.

The present invention has been described in terms of some specific embodiments thereof. It will be appreciated that the embodiments shown in the drawings and described herein are intended for illustrated purposes only and are not by any manner or means intended to be restrictive on the invention. It is believed that the operation and construction of the present invention will be apparent from the foregoing description and drawings appended thereto. It will be clear to the skilled person that the invention is not limited to any embodiment herein described and that modifications are possible which should be considered within the scope of the appended claims. Also kinematic inversions are considered inherently disclosed and to be within the scope of the invention. Moreover, any of the components and elements of the various embodiments disclosed may be combined or may be incorporated in other embodiments where considered necessary, desired or preferred, without departing from the scope of the invention as defined in the claims.

In the claims, any reference signs shall not be construed as limiting the claim. The term 'comprising' and 'including' when used in this description or the appended claims should not be construed in an exclusive or exhaustive sense but rather in an inclusive sense. Thus the expression 'comprising' as used herein does not exclude the presence of other elements or steps in addition to those listed in any claim. Furthermore, the words 'a' and 'an' shall not be construed as limited to 'only one', but instead are used to mean 'at least one', and do not exclude a plurality. Features that are not specifically or explicitly described or claimed may be additionally included in the structure of the invention within its scope. Expressions such as: "means for ..." should be read as: "component configured for ..." or "member constructed to ..." and should be construed to include equivalents for the structures disclosed. The use of expressions like: "critical", "preferred", "especially preferred" etc. is not intended to limit the invention. The invention may be practiced otherwise then as specifically described herein, and is only limited by the appended claims.

## Claims

1. Fetal heart rate monitoring device (1) for monitoring a heart rate of a fetus, the device (1) comprising:
a carrier (3) suitable for being attached to a human body;
at least one fetal electrocardiographic sensor (5) mounted on the carrier (3) and configured for providing an output signal;
at least one reference sensor (13) for providing a reference signal for filtering the output signal to enable identifying a fetal heartrate therefrom;
wherein the device (1) further comprises:
an actuator arrangement comprising a plurality of actuators (10) mounted on the carrier (3) and distributed in an area around the fetal electrocardiographic sensor (5), wherein each actuator (10) is configured for providing a haptic signal (36) to the human body; and
a controller (20) communicatively connected to the at least one fetal electrocardiographic sensor (5) for receiving the output signal, wherein the controller (20) is configured for determining a signal strength from the received output signal, wherein the controller (20) is arranged for controlling each of the plurality of actuators (10) for providing a haptic feedback signal (36) to the human body dependent on the received output signal, wherein the controller (20) is configured for providing the haptic feedback signal (36) dependent on the determined signal strength.

2. Fetal heart rate monitoring device (1) according to claim 1, wherein the controller (20) is configured for determining a heart rate frequency from the received output signal, and for providing the haptic feedback signal (36) as a periodic signal having a periodicity corresponding with the determined heart rate frequency.

3. Fetal heart rate monitoring device (1) according to claim 1, wherein the controller (20) is configured for increasing an intensity of the haptic feedback signal (36) in case the signal strength increases, and for decreasing an intensity of the haptic feedback signal (36) in case the signal strength decreases.

4. Fetal heart rate monitoring device (1) according to claim 1, wherein the controller (20) is configured for individually controlling each of the actuators (10) for providing the haptic feedback signal (36), and for selectively adapting for each respective actuator (10) an intensity of the haptic feedback signal (36) provided via the actuator (10) dependent on the determined signal strength.

5. Fetal heart rate monitoring device (1) according to claim 4, wherein during a relative motion of the carrier (3) on and across the human body, the controller (20) receives one or more signal samples of the output signal and is configured for determining a signal strength from each of the signal samples, and wherein the controller (20) is configured for providing, based on the determined signal strength and by selectively controlling one or more of the actuators (10), an indication of a direction wherein a relative location of the carrier (3) on the human body is to be changed for increasing the determined signal strength.

6. Fetal heart rate monitoring device (1) according to claim 4, wherein the device (1) further comprises one or more auxiliary sensors (12) for providing auxiliary output signals, and wherein the controller (20) is configured for determining an auxiliary signal strength from each of the auxiliary output signals, and for providing, based on the determined auxiliary signal strengths and by selectively controlling one or more of the actuators (10), an indication of a direction wherein a relative location of the carrier (3) on the human body is to be changed for increasing the determined signal strength of the output signal received from the at least one fetal electrocardiographic sensor (5).

7. Fetal heart rate monitoring device (1) according to any one or more of the claims 3-6, wherein the device (1) comprises one of:
at least four actuators (10) distributed in the area around the at least one fetal electrocardiographic sensor (5) such that at least one of the actuator (10) is located in each quarter of the area; or
a plurality of actuators (10) such as to comprise at least n actuators (10), wherein the area around the at least one fetal electrocardiographic sensor (5) comprises an n number of sectors, such that at least one actuator (10) is located in each one of the n number of sectors.

8. Fetal heart rate monitoring device (1) according to any one or more of claims 3-7, wherein the controller (20) is configured for providing, based on the determined signal strength, and where dependent on claim 6 the auxiliary signal strengths, an indication of a relative location of the heart of the fetus in the human body by selectively controlling the haptic feedback signal (36) of one or more of the actuators (10).

9. Fetal heart rate monitoring device (1) according to any one or more of the preceding claims, wherein the carrier comprises a flat bendable or flexible substrate material.

10. Fetal heart rate monitoring device (1) according to any one or more of the preceding claims, wherein at least one of:
the carrier (3) is arranged for being adhered to a skin of the human body;
the carrier (3) is provided by a patch for attachment to the human body;
the carrier (3) is provided by a belt or cloth that may be attached around an abdominal region of the human body;
the carrier (3) is arranged for supporting the fetal electrocardiographic sensor (5) such as to be located on the human body on a belly thereof, optionally in at least one of: an umbilical region, a right or left lumbar region, a hypogastrium region, or a right or left iliac region.

11. Fetal heart rate monitoring device (1) according to any one or more of the preceding claims, wherein one or more of:
the at least one fetal electrocardiographic sensor (5), the reference sensor (13), the actuator arrangement, one or more the actuators (10) of the actuator arrangement, one or more electrical connections of the device (1), or one or more electrodes of the device (1),
are provided by printed electronics.

12. Fetal heart rate monitoring device (1) according to any one or more of the preceding claims, wherein the device (1) further comprises at least one ground electrode located in between at least one of the fetal electrocardiographic sensors (5) and the reference sensor (13).

13. Method of controlling a fetal heart rate monitoring device (1) for monitoring a heart rate of a fetus located inside a human body, wherein the device (1) comprises a carrier (3) suitable for being attached to a human body, a controller (20), at least one fetal electrocardiographic sensor (5) mounted on the carrier (3) and configured for providing an output signal to the controller (20), at least one reference sensor (13) for providing a reference signal for filtering the output signal to enable identifying a fetal heartrate therefrom, and a plurality of actuators (10) mounted on the carrier (3) and distributed in an area around the fetal electrocardiographic sensor (5), wherein each actuator (10) is configured for providing a haptic signal (36) to the human body; the method comprising a steps of:
receiving, by the controller (20), the output signal from the at least one fetal electrocardiographic sensor (5);
determining, by the controller (20), a signal strength from the received output signal, and
controlling, by the controller (20), each of the plurality of actuators (10) for providing a haptic feedback signal (36) to the human body dependent on the received output signal, wherein the haptic feedback signal (36) is provided by the controller (20) dependent on the determined signal strength.

14. Method according to claim 13, further comprising:
obtaining, by the controller (20), at least one of: one or more signal samples of the output signal of the at least one fetal electrocardiographic sensor (5), or one or more signal samples of auxiliary output signals provided by auxiliary sensors (12);
determining, by the controller (20), one or more signals strengths from the signal samples; and
providing, by the controller (20), based on the determined signal strengths and by selectively controlling one or more of the actuators (10), an indication of a direction wherein a relative location of the carrier (3) on the human body is to be changed for increasing a signal strength of the output signal received from the at least one fetal electrocardiographic sensor (5).

15. Method according to any one or more of the claims 13 or 14, further comprising:
obtaining, by the controller (20), at least one of: one or more signal samples of the output signal of the at least one fetal electrocardiographic sensor (5), or one or more signal samples of auxiliary output signals provided by auxiliary sensors (12);
determining, by the controller (20), one or more signals strengths from the signal samples, and an expected relative location of the heart of the fetus in the human body; and
providing an indication of the determined expected relative location of the heart of the fetus in the human body by selectively controlling the haptic signal (36) of one or more of the actuators (10).

## Patentansprüche

1. Vorrichtung (1) zur Überwachung der Herzfrequenz eines Fötus, wobei die Vorrichtung (1) Folgendes umfasst:
einen Träger (3), der an einem menschlichen Körper befestigt werden kann;
mindestens einen fötalen elektrokardiographischen Sensor (5), der auf dem Träger (3) angebracht ist und für die Erbringung eines Ausgangssignals konfiguriert ist;
mindestens einen Referenzsensor (13) zur Erbringung eines Referenzsignals zur Filterung des Ausgangssignals, um daraus eine fötale Herzfrequenz zu ermitteln;
wobei die Vorrichtung (1) weiterhin umfasst:
eine Aktuatoranordnung mit einer Vielzahl von Aktuatoren (10), die auf dem Träger (3) angebracht und in einem Bereich um den fötalen elektrokardiographischen Sensor (5) herum verteilt sind, wobei jeder Aktuator (10) so konfiguriert ist, dass er ein haptisches Signal (36) an den menschlichen Körper erbringt; und
einen Controller (20), der kommunikativ mit dem mindestens einen fötalen elektrokardiographischen Sensor (5) verbunden ist, um das Ausgangssignal zu empfangen, wobei der Controller (20) konfiguriert ist, um eine Signalstärke aus dem empfangenen Ausgangssignal zu bestimmen, wobei der Controller (20) angeordnet ist, um jeden der mehreren Aktuatoren (10) zu steuern, um ein haptisches Rückkopplungssignal (36) für den menschlichen Körper in Abhängigkeit von dem empfangenen Ausgangssignal zu erbringen, wobei der Controller (20) konfiguriert ist, um das haptische Rückkopplungssignal (36) in Abhängigkeit von der bestimmten Signalstärke zu erbringen.

2. Vorrichtung (1) zur Überwachung der Herzfrequenz eines Fötus nach Anspruch 1, wobei der Controller (20) so konfiguriert ist, dass er aus dem empfangenen Ausgangssignal eine Herzfrequenz bestimmt und das haptische Rückkopplungssignal (36) als ein periodisches Signal mit einer Periodizität bereitstellt, die der bestimmten Herzfrequenz entspricht.

3. Vorrichtung (1) zur Überwachung der Herzfrequenz eines Fötus nach Anspruch 1, wobei der Controller (20) so konfiguriert ist, dass er eine Intensität des haptischen Rückkopplungssignals (36) erhöht, wenn die Signalstärke zunimmt, und eine Intensität des haptischen Rückkopplungssignals (36) verringert, wenn die Signalstärke abnimmt.

4. Vorrichtung (1) zur Überwachung der Herzfrequenz eines Fötus nach Anspruch 1, wobei der Controller (20) konfiguriert ist, um jeden der Aktuatoren (10) zur Erbringung des haptischen Rückmeldesignals (36) einzeln zu steuern und für jeden jeweiligen Aktuator (10) eine Intensität des über den Aktuator (10) erbrachten haptischen Rückmeldesignals (36) in Abhängigkeit von der ermittelten Signalstärke selektiv anzupassen.

5. Vorrichtung (1) zur Überwachung der Herzfrequenz eines Fötus nach Anspruch 4, wobei während einer Relativbewegung des Trägers (3) auf und über den menschlichen Körper der Controller (20) einen oder mehrere Signalproben des Ausgangssignals empfängt und konfiguriert ist, um eine Signalstärke aus jedem der Signalproben zu bestimmen, und wobei der Controller (20) konfiguriert ist, um auf der Grundlage der bestimmten Signalstärke und durch selektives Steuern eines oder mehrerer der Aktuatoren (10) einen Hinweis einer Richtung zu erbringen, in der eine relative Position des Trägers (3) auf dem menschlichen Körper zu ändern ist, um die bestimmte Signalstärke zu erhöhen.

6. Vorrichtung (1) zur Überwachung der Herzfrequenz eines Fötus nach Anspruch 4, wobei die Vorrichtung (1) ferner einen oder mehrere Hilfssensoren (12) zum Bereitstellen von Hilfsausgangssignalen umfasst, und wobei der Controller (20) konfiguriert ist, um eine Hilfssignalstärke von jedem der Hilfsausgangssignale zu bestimmen, und um auf der Grundlage der bestimmten Hilfssignalstärken und durch selektives Steuern eines oder mehrerer der Aktuatoren (10) einen Hinweis einer Richtung zu erbringen, in der eine relative Position des Trägers (3) auf dem menschlichen Körper zu ändern ist, um die bestimmte Signalstärke des von dem mindestens einen fötalen elektrokardiographischen Sensor (5) empfangenen Ausgangssignals zu erhöhen.

7. Vorrichtung (1) zur Überwachung der Herzfrequenz eines Fötus nach einem oder mehreren der Ansprüche 3 bis 6, wobei die Vorrichtung (1) eines der folgenden Merkmale umfasst:
mindestens vier Aktuatoren (10), die in dem Bereich um den mindestens einen fötalen elektrokardiographischen Sensor (5) herum verteilt sind, so dass sich mindestens einer der Aktuatoren (10) in jedem Viertel des Bereichs befindet; oder
eine Vielzahl von Aktuatoren (10), so dass sie mindestens n Aktuatoren (10) umfassen, wobei der Bereich um den mindestens einen fötalen elektrokardiographischen Sensor (5) eine n-fache Anzahl von Sektoren umfasst, so dass mindestens ein Aktuator (10) in jedem der n-fachen Anzahl von Sektoren angeordnet ist.

8. Vorrichtung (1) zur Überwachung der Herzfrequenz eines Fötus nach einem oder mehreren der Ansprüche 3 bis 7, wobei der Controller (20) so konfiguriert ist, dass er auf der Grundlage der ermittelten Signalstärke und, falls dies von Anspruch 6 abhängt, der Stärken des Hilfssignals, einen Hinweis der relativen Position des Herzens des Fötus im menschlichen Körper erbringt, indem er das haptische Rückkopplungssignal (36) eines oder mehrerer der Aktuatoren (10) selektiv steuert.

9. Vorrichtung (1) zur Überwachung der Herzfrequenz eines Fötus nach einem oder mehreren der vorhergehenden Ansprüche, wobei der Träger ein flaches, biegsames oder flexibles Substratmaterial umfasst.

10. Vorrichtung (1) zur Überwachung der Herzfrequenz eines Fötus nach einem oder mehreren der vorangehenden Ansprüche, wobei mindestens eine der folgenden Bedingungen erfüllt ist:
der Träger (3) ist so beschaffen, dass er auf die Haut des menschlichen Körpers aufgeklebt werden kann;
der Träger (3) ist mit einem Pflaster zur Befestigung am menschlichen Körper versehen;
der Träger (3) ist mit einem Gürtel oder einem Tuch versehen, das um den Bauchbereich des menschlichen Körpers befestigt werden kann;
der Träger (3) ist so angeordnet, dass er den fötalen elektrokardiographischen Sensor (5) so trägt, dass er sich am menschlichen Körper auf dessen Bauch befindet, wahlweise in mindestens einem der folgenden Gegenden: eine Nabelgegend, eine rechte oder linke Lendengegend, eine Unterbauchgegend oder eine rechte oder linke Iliakalgegend.

11. Vorrichtung (1) zur Überwachung der Herzfrequenz eines Fötus nach einem oder mehreren der vorhergehenden Ansprüche, wobei einer oder mehrere von:
dem mindestens einen fötalen elektrokardiographischen Sensor (5), dem Referenzsensor (13), der Aktuatoranordnung, einem oder mehreren Aktuatoren (10) der Aktuatoranordnung, einem oder mehreren elektrischen Anschlüssen der Vorrichtung (1) oder einer oder mehreren Elektroden der Vorrichtung (1)
durch gedruckte Elektronik bereitgestellt werden.

12. Vorrichtung (1) zur Überwachung der Herzfrequenz eines Fötus nach einem oder mehreren der vorhergehenden Ansprüche, wobei die Vorrichtung (1) ferner mindestens eine Erdungselektrode umfasst, die sich zwischen mindestens einem der fötalen elektrokardiographischen Sensoren (5) und dem Referenzsensor (13) befindet.

13. Verfahren zur Steuerung einer Vorrichtung zur Überwachung der Herzfrequenz eines Fötus (1) zur Überwachung der Herzfrequenz eines im menschlichen Körper befindlichen Fötus, wobei die Vorrichtung (1) umfasst:
einen Träger (3), der an einem menschlichen Körper befestigt werden kann,
einen Controller (20),
mindestens einen fötalen elektrokardiographischen Sensor (5), der auf dem Träger (3) angebracht ist und der so konfiguriert ist, dass er ein Ausgangssignal an den Controller (20) bereitstellt,
mindestens einen Referenzsensor (13) zum Bereitstellen eines Referenzsignals zum Filtern des Ausgangssignals, um daraus eine Herzfrequenz eine Fötus identifizieren zu können, und
eine Vielzahl von Aktuatoren (10), die auf dem Träger (3) angebracht und in einem Bereich um den fötalen elektrokardiographischen Sensor (5) herum verteilt sind,
wobei jeder Aktuator (10) so konfiguriert ist, dass er ein haptisches Signal (36) an den menschlichen Körper erbringt; wobei das Verfahren die folgenden Schritte umfasst:
Empfangen des Ausgangssignals von dem mindestens einen fötalen elektrokardiographischen Sensor (5) durch den Controller (20);
Bestimmen einer Signalstärke aus dem empfangenen Ausgangssignal durch den Controller (20), und
Steuerung durch den Controller (20) jedes der Vielzahl der Aktuatoren (10) zur Erbringung eines haptischen Rückkopplungssignals (36) an den menschlichen Körper in Abhängigkeit von dem empfangenen Ausgangssignal, wobei das haptische Rückkopplungssignal (36) vom dem Controller (20) in Abhängigkeit von der ermittelten Signalstärke erbracht wird.

14. Verfahren nach Anspruch 13, welches ferner umfasst:
Erlangen mindestens einer der folgenden Informationen durch den Controller (20): eine oder mehrere Signalproben des Ausgangssignals des mindestens einen fötalen elektrokardiographischen Sensors (5) oder eine oder mehrere Signalproben von Hilfsausgangssignalen, die von Hilfssensoren (12) bereitgestellt werden;
Bestimmen einer oder mehrerer Signalstärken aus den Signalproben durch den Controller (20); und
Erbringen durch den Controller (20) eines Hinweises einer Richtung, in der eine relative Position des Trägers (3) auf dem menschlichen Körper geändert werden soll, um eine Signalstärke des von dem mindestens einen fötalen elektrokardiographischen Sensor (5) empfangenen Ausgangssignals zu erhöhen, auf der Grundlage der bestimmten Signalstärken und durch selektives Steuern eines oder mehrerer der Aktuatoren (10).

15. Verfahren nach einem oder mehreren der Ansprüche 13 oder 14, welches ferner umfasst:
Erlangen mindestens einer der folgenden Informationen durch den Controller (20): eine oder mehrere Signalproben des Ausgangssignals des mindestens einen fötalen elektrokardiographischen Sensors (5) oder eine oder mehrere Signalproben von Hilfsausgangssignalen, die von Hilfssensoren (12) bereitgestellt werden;
Bestimmen einer oder mehrerer Signalstärken aus den Signalproben und einer erwarteten relativen Lage des Herzens des Fötus im menschlichen Körper durch den Controller (20); und
Erbringen eines Hinweises der ermittelten erwarteten relativen Lage des Herzens des Fötus im menschlichen Körper durch selektive Steuerung des haptischen Signals (36) eines oder mehrerer der Aktuatoren (10).

## Revendications

1. Dispositif de surveillance de rythme cardiaque fœtal (1) pour surveiller le rythme cardiaque d'un fœtus, le dispositif (1) comprenant :
un support (3) adapté pour être fixé à un corps humain ;
au moins un capteur électrocardiographique fœtal (5) monté sur le support (3) et configuré pour fournir un signal de sortie ;
au moins un capteur de référence (13) pour fournir un signal de référence pour filtrer le signal de sortie afin de permettre une identification d'un appareil cardiaque fœtal à partir de celui-ci ;
dans lequel le dispositif (1) comprend en outre :
un agencement d'actionnement comprenant une pluralité d'actionneurs (10) montés sur le support (3) et répartis dans une zone autour du capteur électrocardiographique fœtal (5), dans lequel chaque actionneur (10) est configuré pour fournir un signal haptique (36) au corps humain ; et
un dispositif de commande (20) relié en communication au au moins un capteur électrocardiographique fœtal (5) pour recevoir le signal de sortie, dans lequel le dispositif de commande (20) est configuré pour déterminer une intensité de signal à partir du signal de sortie reçu, dans lequel le dispositif de commande (20) est agencé pour commander chacun de la pluralité d'actionneurs (10) afin de fournir un signal de rétroaction haptique (36) au corps humain en fonction du signal de sortie reçu, dans lequel le dispositif de commande (20) est configuré pour fournir le signal de rétroaction haptique (36) en fonction de l'intensité de signal déterminée.

2. Dispositif de surveillance de rythme cardiaque fœtal (1) selon la revendication 1, dans lequel le dispositif de commande (20) est configuré pour déterminer une fréquence de rythme cardiaque à partir du signal de sortie reçu, et pour fournir le signal de rétroaction haptique (36) en tant que signal périodique présentant une périodicité correspondant à la fréquence de rythme cardiaque déterminée.

3. Dispositif de surveillance de rythme cardiaque fœtal (1) selon la revendication 1, dans lequel le dispositif de commande (20) est configuré pour augmenter une intensité du signal de rétroaction haptique (36) dans un cas où l'intensité de signal augmente, et pour diminuer une intensité du signal de rétroaction haptique (36) dans un cas où l'intensité de signal diminue.

4. Dispositif de surveillance de rythme cardiaque fœtal (1) selon la revendication 1, dans lequel le dispositif de commande (20) est configuré pour commander individuellement chacun des actionneurs (10) pour fournir le signal de rétroaction haptique (36), et pour adapter sélectivement pour chaque actionneur respectif (10) une intensité du signal de rétroaction haptique (36) fourni via l'actionneur (10) en fonction de l'intensité de signal déterminée.

5. Dispositif de surveillance de rythme cardiaque fœtal (1) selon la revendication 4, dans lequel pendant un mouvement relatif du support (3) sur et à travers le corps humain, le dispositif de commande (20) reçoit un ou plusieurs échantillons de signal du signal de sortie et est configuré pour déterminer une intensité de signal à partir de chacun des échantillons de signal, et dans lequel le dispositif de commande (20) est configuré pour fournir, sur la base de l'intensité de signal déterminée et en commandant sélectivement un ou plusieurs des actionneurs (10), une indication d'une direction dans laquelle un emplacement relatif du support (3) sur le corps humain doit être modifié pour augmenter l'intensité de signal déterminée.

6. Dispositif de surveillance de rythme cardiaque fœtal (1) selon la revendication 4, dans lequel le dispositif (1) comprend en outre un ou plusieurs capteurs auxiliaires (12) pour fournir des signaux de sortie auxiliaires, et dans lequel le dispositif de commande (20) est configuré pour déterminer une intensité de signal auxiliaire à partir de chacun des signaux de sortie auxiliaires, et pour fournir, sur la base des intensités de signal auxiliaires déterminées et en commandant sélectivement un ou plusieurs des actionneurs (10), une indication d'une direction dans laquelle un emplacement relatif du support (3) sur le corps humain doit être modifié pour augmenter l'intensité de signal déterminée du signal de sortie reçu à partir du au moins un capteur électrocardiographique fœtal (5).

7. Dispositif de surveillance de rythme cardiaque fœtal (1) selon une quelconque des revendications 3 à 6, dans lequel le dispositif (1) comprend l'un des éléments suivants :
au moins quatre actionneurs (10) répartis dans la zone autour du au moins un capteur électrocardiographique fœtal (5) de telle sorte qu'au moins un des actionneurs (10) soit situé dans chaque quartier de la zone ; ou
une pluralité d'actionneurs (10) de manière à comprendre au moins n actionneurs (10), dans lequel la zone autour du au moins un capteur électrocardiographique fœtal (5) comprend un nombre n de secteurs, de telle sorte qu'au moins un actionneur (10) est situé dans chacun du nombre n de secteurs.

8. Dispositif de surveillance de rythme cardiaque fœtal (1) selon une quelconque ou plusieurs des revendications 3 à 7, dans lequel le dispositif de commande (20) est configuré pour fournir, sur la base de l'intensité de signal déterminée, et lorsque cela dépend de la revendication 6, des intensités de signal auxiliaires, une indication d'un emplacement relatif du cœur du fœtus dans le corps humain en commandant sélectivement le signal de rétroaction haptique (36) d'un ou plusieurs des actionneurs (10).

9. Dispositif de surveillance de rythme cardiaque fœtal (1) selon une quelconque ou plusieurs des revendications précédentes, dans lequel le support comprend un matériau de substrat pliable ou flexible plat.

10. Dispositif de surveillance de rythme cardiaque fœtal (1) selon une quelconque ou plusieurs des revendications précédentes, dans lequel :
le support (3) est agencé pour être mis en adhérence sur une peau du corps humain ;
le support (3) est fourni par un timbre destiné à une fixation sur le corps humain ;
le support (3) est fourni par une ceinture ou un tissu qui peut être fixé(e) autour d'une région abdominale du corps humain ;
le support (3) est agencé pour supporter le capteur électrocardiographique fœtal (5) de manière à être situé sur le corps humain sur un ventre de celui-ci, facultativement dans au moins une parmi : une région ombilicale, une région lombaire droite ou gauche, une région hypogastrique, ou une région iliaque droite ou gauche.

11. Dispositif de surveillance de rythme cardiaque fœtal (1) selon l'une quelconque des revendications précédentes, dans lequel un ou plusieurs des éléments suivants :
au moins un capteur électrocardiographique fœtal (5), le capteur de référence (13), l'agencement d'actionnement, un ou plusieurs actionneurs (10) de l'agencement d'actionnement, une ou plusieurs connexions électriques du dispositif (1), ou une ou plusieurs électrodes du dispositif (1),
sont fournis par de l'électronique imprimée.

12. Dispositif de surveillance de rythme cardiaque fœtal (1) selon une quelconque ou plusieurs des revendications précédentes, dans lequel le dispositif (1) comprend en outre au moins une électrode de masse située entre au moins un des capteurs électrocardiographiques fœtaux (5) et le capteur de référence (13).

13. Procédé de commande d'un dispositif de surveillance de rythme cardiaque fœtal (1) pour surveiller le rythme cardiaque d'un fœtus situé à l'intérieur d'un corps humain, dans lequel le dispositif (1) comprend : un support (3) approprié pour être fixé à un corps humain, un dispositif de commande (20), au moins un capteur électrocardiographique fœtal (5) monté sur le support (3) et configuré pour fournir un signal de sortie au dispositif de commande (20), au moins un capteur de référence (13) pour fournir un signal de référence pour filtrer le signal de sortie afin de permettre une identification d'un rythme cardiaque fœtal à partir de celui-ci, et une pluralité d'actionneurs (10) montés sur le support (3) et répartis dans une zone autour du capteur électrocardiographique fœtal (5), dans lequel chaque actionneur (10) est configuré pour fournir un signal haptique (36) au corps humain ; le procédé comprenant les étapes consistant à :
recevoir, par l'intermédiaire du dispositif de commande (20), le signal de sortie provenant du au moins un capteur électrocardiographique fœtal (5) ;
déterminer, par l'intermédiaire du dispositif de commande (20), une intensité de signal à partir du signal de sortie reçu, et
commander, par l'intermédiaire du dispositif de commande (20), chacun de la pluralité d'actionneurs (10) pour fournir un signal de rétroaction haptique (36) au corps humain en fonction du signal de sortie reçu,
dans lequel le signal de rétroaction haptique (36) est fourni par le dispositif de commande (20) en fonction de l'intensité de signal déterminée.

14. Procédé selon la revendication 13, comprenant en outre les étapes consistant à
obtenir, par l'intermédiaire du dispositif de commande (20), au moins un parmi : un ou plusieurs échantillons de signal du signal de sortie du au moins un capteur électrocardiographique fœtal (5), ou un ou plusieurs échantillons de signal de signaux de sortie auxiliaires fournis par des capteurs auxiliaires (12) ;
déterminer, par l'intermédiaire du dispositif de commande (20), une ou plusieurs intensités de signaux à partir des échantillons de signal ; et
fournir, par l'intermédiaire du dispositif de commande (20), sur la base des intensités de signal déterminées et en commandant sélectivement un ou plusieurs des actionneurs (10), une indication d'une direction dans laquelle un emplacement relatif du support (3) sur le corps humain doit être modifié pour augmenter une intensité de signal du signal de sortie reçu à partir du au moins un capteur électrocardiographique fœtal (5).

15. Procédé selon une quelconque ou plusieurs des revendications 13 ou 14, comprenant en outre les étapes consistant à :
obtenir, par l'intermédiaire du dispositif de commande (20), au moins un parmi : un ou plusieurs échantillons de signal du signal de sortie du au moins un capteur électrocardiographique fœtal (5), ou un ou plusieurs échantillons de signal de signaux de sortie auxiliaires fournis par des capteurs auxiliaires (12) ;
déterminer, par l'intermédiaire du dispositif de commande (20), une ou plusieurs intensités de signal à partir des échantillons de signal, et un emplacement relatif attendu du cœur du fœtus dans le corps humain ; et
fournir une indication de la position relative attendue déterminée du cœur du fœtus dans le corps humain en commandant sélectivement le signal haptique (36) d'un ou plusieurs des actionneurs (10).
